# EUROPEAN PATENT APPLICATION

(11) **EP 1 974 764 A1**
(43) Date of publication of application: **01.10.2008**
(21) Application number: 08153008.1
(22) Date of filing: 19.03.2008
(51) Int. Cl.: A61M 16/10

(54) **Method in a patient ventilating system**

(30) Priority: 29.03.2007 US 729523
(71) Applicant: GENERAL ELECTRIC COMPANY, Schenectady, NY 12345 (US)
(72) Inventor: Rantala, Borje, 00670 Helsinki (FI); Paloheimo, Markku, 02320 Espoo (FI)
(74) Representative: Illingworth-Law, William Illingworth

(57) **Abstract**

Method in a patient ventilating system to be used for ex-ample in awakening a patient at the end stage of an anesthesia procedure in which a carestation (1) comprising an anesthesia machine (3) is used. The method comprises the steps of setting a target value for the patient end tidal CO2 (EtCO2), and performing increase of the patient end tidal CO2 to the set target value by automatically using a ventilator in the anesthesia machine, and keeping blood oxygenation continuously at a safe level.

## Description

The invention relates to a method of awakening of a patient the end stage of an anesthesia procedure, i.e. for example in the procedure where for example a carestation comprising an anesthesia machine is used.

When an anesthesia procedure of a patient is moving towards the end for example after a surgical operation it is advantageous to increase the patient end tidal CO2 (EtCO2), so as to increase respiratory drive, force him/her to wake up and to start breathing. This is usually done manually by an anaesthesiologist by reducing the minute ventilation (MV).

Medical literature widely known in the field describes several reports of ventilator designs using feedback control of the minute ventilation to achieve a set patient end tidal.

It is known in the field that there are several issues to be observed when reducing the minute ventilation, for example what is the present patient end tidal CO2 value and what is the value of the present minute ventilation. Further it must be known whether or not said values have been stable for a few minutes. When waking the patient up, the metabolic rate is expected to increase, and thus the patient is expected to need more minute ventilation in order to keep the patient end tidal CO2 constant. This is especially true if the patient in question has been relaxed by neuromuscular blockers.

Drawbacks of the known prior art relate to human factors, i.e. methods used previously are based on the level of skills of the personnel and the circumstances in which decisions to control the process must be made. It must be noted that a person who is in charge can fore some reason be tired or the circumstances may be somehow irritating and therefore the ability of the person in charge to make a correct solution may me restricted. It must be seen further that a person in charge may be for example young having relative restricted experience, and therefore his/her ability to make correct decisions is restricted when compared to a person having tens of years of practical working experience.

In other words the results obtained by using the techniques known in the prior art is greatly affected by the skills of the person in charge and the circumstances occurring at the time when the decisions must be made. These human factors may lead to decreases in the patient safety level. This cannot be approved because for example in the United States mistakes may lead to extremely high financial compensations.

The object of the invention is to obtain a method by which as even circumstances as possible can be achieved as regards patient safety irrespective of the working experience of the person in charge and irrespective the circumstances at the moment the decisions must be made. This is obtained with the present invention. The present invention is basically characterized in that the method comprises the following steps; setting a target value for the patient end tidal (EtCO2) and performing increase of the patient end tidal CO2 to the set target value by automatically using a ventilator in the anesthesia machine, and keeping blood oxygenation continuously at a safe level.

The advantage of the invention is that risks of mistakes in the patient care is lower when compared to the traditional manual methods, i.e. the invention offer a reliable way for awakening of a patient so that effects of the working experience of the persons in charge and the circumstanced occurring at the event in question are minimized.

The invention will be described in greater detail by way of edample only by means of an embodiment described in the attached drawing, whereby

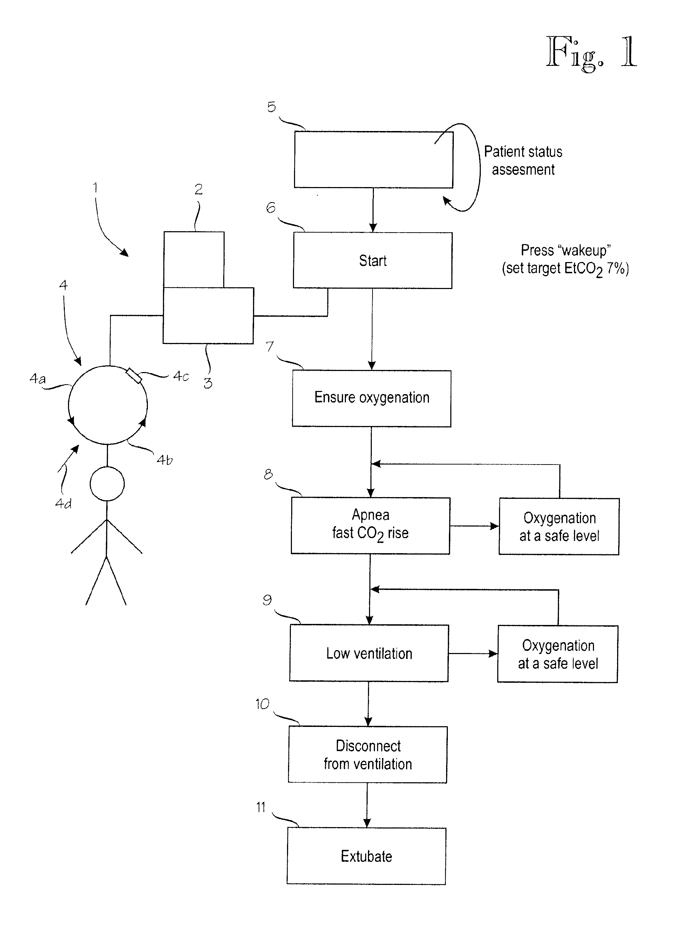

Figure 1 shows a block schema describing one embodiment of the invention.

The present invention relates to a method of awakening a patient at the end stage of an anesthesia procedure in which a carestation 1 is used. As told above in the prior art an anaesthesiologist has done awakening of a patient manually by reducing the minute ventilation (MV). The present invention specifies a method in which awakening of the patient is carried out automatically by using a carestation 1, i.e. the carestation 1 is used to perform automatic awakening of the patient.

Figure 1 shows a block schema describing one embodiment of the method of the invention. The embodiment shown in Figure 1 shows a carestation 1 which is provided with a combination of a monitor 2 and an anesthesia machine 3. The carestation 1 can be connected to the patient by using for example a circle type breathing circuit 4 comprising channels 4a, 4b fro inspired and expired air, CO2 absorber 4c, and other accessories enabling the use of the carestation. Said matters are quite familiar to a person skilled in the art and therefore said matters are not described here.

The first box 5 in Figure 1 shows a present patient status assessment step in which for example metabolic state and oxygenation level of the patient is defined. This procedure is carried out before the actual wakeup sequence is started. This procedure includes also monitoring of for example if the patient is paralysed by neuromuscular blockers or like at the moment the wakeup sequence is started. Said neuromuscular blockers lead to additional corrections in the wakeup procedure. Increased metabolic activity of waking up must also be taken account in the procedure.

According to the basic idea of the invention the first step is to set the target value for the end tidal CO2 (EtCO2). The second step is to increase the end tidal CO2 (EtCO2) to the set target value by automatically using a ventilator in the anesthesia machine, for example to reduce the minute ventilation, and keeping blood oxygenation of the patient at a safe level.

In other words when the target value for the end tidal CO*2 is set, for example the end tidal CO2 value is set to rise from 5% to 7% then the anesthesia machine, after a start button is pressed, automatically increase the end tidal CO2 to the set value. Box 6 in Figure 1 describes said step. Blood oxygenation is also kept adequate, i.e. at a safe level during said process by preoxygenating the patient with high inspiratory oxygen before the reduction in ventilation begins as described in box 7.

To control said increase of the end tidal CO2 (EtCO2) it is also advantageous to set an appropriate ramp time for the target value for the end tidal CO2. For a fast ramp time, the carestation 1 can compute the minute ventilation necessary to get to the EtCO2 target value, or for fastest ramptime, set an apnea period with zero ventilation. For longer ramp times, a gradual change, i.e. a reduction in tidal volume or respiratory rate or change in both, is more appropriate. The apneic period may be interrupted by occasional breath to enable assessment of the EtCO2. The ramp time may be either linear or nonlinear, and can start with an apneic period. Assessment of the blood oxygenation is continuously performed by pulse oximetry during the time of reduced ventilation. This step is described in box 8.

As described above according to the invention it is essential that a person in charge, for example a clinician, selects the target EtCO2 value and appropriate ramp time, and then the algorithm accepts said selection or alternatively informs that said selection is inappropriate. If the selection is appropriate then the algorithm carries out the awakening period by automatically controlling the carestation 1 as shown in Figure 1.

As described above increase of the patient end tidal CO2 can be performed by reducing the minute ventilation (MV). This can be done either by reducing the tidal volume (TV) or by reducing the respiratory rate (RR). It is also possible to use a combination of the two alternatives described above. The algorithm controls a ventilator in the anesthesia machine 3 automatically so that the target value of EtCO2 is achieved with a controlled ramp time. Said ramp time can be preselected as described above. During the procedure there are naturally safety checks so that the procedure can be carried out correctly and safely and so that any dangerous and harmful circumstances can be noticed in time. The algorithm gives an appropriate alarm in said circumstances.

When the end tidal CO2 is increased it is possible to reduce the minute ventilation by a preset ramp time or an apneic period. The combination of the ramp time and apneic period can also be used. The apneic period can also be adjusted in order to take account the increased metabolic activity of waking up, and further if reducing of the respiratory rate is used to reduce the minute ventilation, the reduction of the respiratory rate can be adjusted in order to take account the increased metabolic activity of waking up. This step is described in box 9.

When the target EtCO2 value has been obtained and all safety demands have been fulfilled the patient can be disconnected from ventilation as described in box 10. After disconnection extubation can be carried out as shown as described in box 11 in Figure 1.

The increase of the patient and tidal CO2 can also be performed by bypassing the CO2 absorber 4c in the circle type patient breathing circuit 4. It is also possible to increase patient end tidal CO2 by injecting CO2 into the patient breathing system as described schematically by an arrow 4d in figure 1.

The invention has been described here in connection with a carestation. The invention is however nor restricted to the embodiment but described in Figure 1 but the invention can be understood wider, i.e. so that the invention can also be used in connection with a patient ventilating system. In other words within the spirit of the invention the method described can be used so that a target value for the patient end tidal CO2 (EtCO2) is set, and patient respiratory drive is stimulated by using the ventilator automatically so that the patient end tidal CO2 is increased to the preset target value level in the way described above.

## Claims

1. Method of awakening a patient at the end stage of an anesthesia procedure in which a carestation (1) comprising an anesthesia machine (3) is used, **characterized in that** the method comprises following steps;
setting a target value for the patient end tidal CO2 (EtCO2), and
performing increase of the patient end tidal CO2 to the set target value by automatically using a ventilator in the anesthesia machine (3), and keeping blood oxygenation continuously at a safe level.

2. The method of claim 1, **characterized in that** the increase of the patient end tidal CO2 is performed by reducing the minute ventilation.

3. The method of claim 1 or claim 2, **characterized in that** the method further comprises a step for setting a ramp time for the target value for the patient end tidal CO2.

4. The method of claim 2, **characterized in that** reducing of the minute ventilation is performed by reducing the tidal volume.

5. The method of claim 2, **characterized in that** reducing of the minute ventilation is performed by reducing the respiratory rate.

6. The method of claim 2, **characterized in that** increasing of the patient end tidal CO2 is performed by reducing the minute ventilation by a preset apneic period.

7. The method of claim 6, **characterized in that** increasing of the patient end tidal CO2 is performed by combining an apneic period with a controlled ramp time, wherein the apneic period is adjusted in order to take into account the increased metabolic activity of waking up.

8. The method of claim 5, **characterized in that** the reduction of the respiratory rate is adjusted in order to take into account the in-creased metabolic activity of waking up.

9. The method of any one of the preceding claims, **characterized in that** the increase of the patient end tidal CO2 is performed by bypassing a CO2 absorber (4c) in the patient breathing circuit (4).

10. The method of any one of the preceding claims, **characterized in that** the increase of the patient end tidal CO2 is performed by injecting CO2 to the patient breathing circuit (4).
